Europäisches Patentamt

European Patent Office

Office européen des brevets

(19)

(11) **EP 1 230 204 B1**

(12) **EUROPÄISCHE PATENTSCHRIFT**

(45) Veröffentlichungstag und Bekanntmachung des Hinweises auf die Patenterteilung:
**13.10.2004 Patentblatt 2004/42**

(21) Anmeldenummer: **00975980.4**

(22) Anmeldetag: **31.10.2000**

(51) Int Cl.$^7$: **C07C 51/25**, C07C 57/04

(86) Internationale Anmeldenummer:
**PCT/EP2000/010771**

(87) Internationale Veröffentlichungsnummer:
**WO 2001/036364 (25.05.2001 Gazette 2001/21)**

(54) **VERFAHREN DER KATALYTISCHEN GASPHASENOXIDATION VON PROPEN ZU ACRYLSÄURE**

METHOD FOR CARRYING OUT THE CATALYTIC GAS PHASE OXIDATION OF PROPENE TO FORM ACRYLIC ACID

PROCEDE POUR L'OXYDATION CATALYTIQUE EN PHASE GAZEUSE DE PROPENE POUR FORMER DE L'ACIDE ACRYLIQUE

(84) Benannte Vertragsstaaten:
**BE DE FR NL**

(30) Priorität: **16.11.1999 DE 19955168**

(43) Veröffentlichungstag der Anmeldung:
**14.08.2002 Patentblatt 2002/33**

(73) Patentinhaber: **BASF AKTIENGESELLSCHAFT**
**67056 Ludwigshafen (DE)**

(72) Erfinder:
• **TENTEN, Andreas**
**67487 Maikammer (DE)**

• **HIBST, Hartmut**
**69198 Schriesheim (DE)**
• **MACHHAMMER, Otto**
**68163 Mannheim (DE)**
• **HECHLER, Claus**
**67063 Ludwigshafen (DE)**
• **MÜLLER-ENGEL, Klaus, Joachim**
**76297 Stutensee (DE)**
• **UNVERRICHT, Signe**
**68169 Mannheim (DE)**

(56) Entgegenhaltungen:
**EP-A- 0 293 224        EP-A- 0 900 774**
**WO-A-00/53558        DE-A- 2 513 405**

**Beschreibung**

Vorliegende Erfindung betrifft ein Verfahren der katalytischen Gasphasenoxidation von Propen zu Acrylsäure, bei dem man ein Propen, molekularen Sauerstoff und wenigstens ein Inertgas enthaltendes Reaktionsgasausgangsgemisch, das den molekularen Sauerstoff und das Propen in einem molaren Verhältnis $O_2: C_3H_6 \geq 1$ enthält in einer ersten Reaktionsstufe unter Anwendung einer Propenbelastung von $\geq 160$ Nl Propen/l Festbettkatalysatorschüttung $1 \cdot h$ so über eine Festbettkatalysatorschüttung 1, die in zwei räumlich aufeinanderfolgenden Reaktionszonen A, B angeordnet ist, wobei die Temperatur der Reaktionszone A 300 bis 370°C und die Temperatur der Reaktionszone B 305 bis 380°C beträgt und gleichzeitig wenigstens 5°C oberhalb der Temperatur der Reaktionszone A liegt und deren Aktivmasse wenigstens ein wenigstens die Elemente Mo, Fe und Bi enthaltendes Multimetalloxid ist, führt, daß sich die Reaktionszone A bis zu einem Umsatz des Propens von 40 bis 80 mol-% erstreckt und der Propenumsatz bei einmaligem Durchgang durch die Festbettkatalysatorschüttung $1 \geq 90$ mol-% und die damit einhergehende Selektivität der Acroleinbildung sowie der Acrylsäurenebenproduktbildung zusammengenommen $\geq 90$ mol-% betragen, und das dabei resultierende Produktgasgemisch, das den molekularen Sauerstoff und das Acrolein in einem molaren Verhältnis $O_2: C_3H_4O \geq 0{,}5$ enthält in einer zweiten Reaktionsstufe so über eine Festbettkatalysatorschüttung 2, die entweder in einer einzigen Reaktionszone C oder in zwei räumlich aufeinanderfolgenden Reaktionszonen D, E angeordnet ist, wobei die Temperatur der Reaktionszone C 230 bis 300°C und die Temperatur der Reaktionszone D 230 bis 280°C und die Temperatur der Reaktionszone E 250 bis 300°C beträgt und gleichzeitig wenigstens 5°C oberhalb der Temperatur der Reaktionszone D liegt und die Aktivmasse wenigstens ein wenigstens die Elemente Mo und V enthaltendes Multimetalloxid ist, führt, daß der Acroleinumsatz bei einmaligem Durchgang durch die Reaktionszone C bzw. die Reaktionszonen D und E $\geq 90$ mol-% und die Selektivität der über alle Reaktionszonen bilanzierten Acrylsäurebildung, bezogen auf umgesetztes Propen, $\geq 80$ mol-% beträgt, wobei die zeitliche Abfolge, in der das Reaktionsgasausgangsgemisch die Reaktionszonen durchströmt, der alphabetischen Abfolge der Reaktionszonen entspricht.

Acrylsäure ist ein bedeutendes Monomer, das als solches oder in Form seiner Alkylester zur Erzeugung von z.B. als Klebstoffen geeigneten Polymerisaten Verwendung findet.

Die Herstellung von Acrylsäure kann z.B. durch zweistufige katalytische Gasphasenoxidation von Propen zu Acrylsäure erfolgen.

Das Verfahren der zweistufigen katalytischen Gasphasenoxidation von Propen zu Acrylsäure ist allgemein bekannt (vgl. z.B. die DE-A 3002829). Im besonderen sind die beiden Reaktionsstufen für sich bekannt (vgl. z.B. EP-A 714700, EP-A 700893, EP-A 15565, DE-C 2830765, DE-C 3338380, JP-A 91/294239, EP-A 807465, WO 98/24746, EP-B 279374, DE-C 2513405, DE-A 3300044, EP-A 575897 und DE-A 19855913).

Im besonderen wurde schon vorgeschlagen, die beiden Reaktionsstufen in zwei je zwei Temperaturzonen aufweisenden Vielkontaktrohrbündelreaktoren zu realisieren (vgl. z.B. DE-A 19948241, DE-A 19948523, DE-A 19910506, DE-A 19910508 und DE-A 19948248), was insbesondere im Fall einer hohen Eduktbelastung als vorteilhaft erachtet wird.

Dabei wird in allen vorgenannten Fällen empfohlen, je Reaktionsstufe einen Vielkontaktrohr-Rohrbündelreaktor einzusetzen, d.h., das Verfahren insgesamt in zwei räumlich voneinander getrennten, hintereinander angeordneten Rohrbündelreaktoren durchzuführen.

Hintergrund für diese Empfehlung ist, daß z.B. aus der US-A 4029636 bekannt ist, daß sich aus dem Multimetalloxidkatalysator der bei der höheren Temperatur betriebenen ersten Reaktionsstufe $MoO_3$ verflüchtigt, das sich in der Festbettkatalysatorschüttung 2 der die niedrigere Betriebstemperatur aufweisenden zweiten Reaktionsstufe teilweise wieder abscheidet.

Als Konsequenz resultiert über die Zeit ein zunehmender Druckverlust des die beiden Festbettkatalysatorschüttungen durchströmenden Reaktionsgasgemisches (die Festbettkatalysatorschüttung 2 wächst langsam zu).

Gemäß der EP-A 614 872 wird der sich über die Zeit einstellende Druckverlust noch dadurch verstärkt, daß es in der Festbettkatalysatorschüttung 2 zusätzlich regelmäßig zur Abscheidung organischer Materialien, z.B. von festem Kohlenstoff, kommt.

Abhilfe für das beschriebene Problem ist gemäß der US-A 4029639 dann möglich, wenn man die beiden Reaktionsstufen in zwei räumlich getrennten, hintereinander angeordneten Rohrbündelreaktoren verwirklicht und den Großteil der vorgenannten Abscheidungen in einem zwischen den beiden Rohrbündelreaktoren angebrachten Zwischenkühler vorgenommen wird, der mit inerten Festkörpern bestückt ist.

Die Durchführung der zweistufigen katalytischen Gasphasenoxidation von Propen zu Acrylsäure in einem einzigen Rohrbündelreaktor empfehlen z.B die DE-A 2830765 und die EP-A 911313. Dabei liegt jedoch die Propenbelastung der Festbettkatalysatorschüttung 1 in allen beispielhaften Ausführungsformen bei Werten $\leq 100$ Nl Propen/l Festbettkatalysatorschüttung $1 \cdot h$. Geringere Propenbelastungen sind jedoch gleichbedeutend mit einer geringeren $MoO_3$-Verflüchtigung, da z.B. die innerhalb eines gewissen Zeitraums gebildete Menge an Reaktionswasser (vergleichbare Umsätze vorausgesetzt) bei geringerer Belastung kleiner als bei hoher Belastung ist. Aus der US-A 4029636 ist jedoch bekannt, daß insbesondere Wasserdampf die $MoO_3$-Verflüchtigung fördert.

Zum anderen kann sich bei einer höheren Propenlast pro Stunde eine größere Menge $MoO_3$ verflüchtigen, da naturgemäß einer größeren Gasmenge eine größere $MoO_3$-Sättigungsmenge entspricht.

Auf der anderen Seite ist eine Verwirklichung der zweistufigen gasphasenkatalytisch oxidativen Festbettoxidation von Propen zur Herstellung von Acrylsäure in zwei räumlich getrennten Vielkontaktrohr-Rohrbündelreaktoren besonders investitionsintensiv.

Die Aufgabe der vorliegenden Erfindung bestand daher darin, ein Verfahren der zweistufigen gasphasenkatalytisch oxidativen Festbettoxidativen von Propen zu Acrylsäure in einem einzigen Vielkontaktrohr-Rohrbündelreaktor zur Verfügung zu stellen, und dies bei erhöhter Propenbelastung der Festbettkatalysatorschüttung 1, das den Nachteil eines über die Zeit rasch anwachsenden Druckverlustes längs der Kontaktrohre nur noch in geminderter Form aufweist.

Überraschend wurde nun gefunden, daß dies dann möglich ist, wenn man die erste Reaktionsstufe, wie eingangs beschrieben, in zwei räumlich aufeinanderfolgenden Temperaturzonen A, B verwirklicht.

Gegenstand der vorliegenden Erfindung ist somit ein Verfahren der katalytischen Gasphasenoxidation von Propen zu Acrylsäure, bei dem man ein Propen, molekularen Sauerstoff und wenigstens ein Inertgas enthaltendes Reaktionsgasausgangsgemisch, das den molekularen Sauerstoff und das Propen in einem molaren Verhältnis $O_2$: $C_3H_6 \geq 1$ enthält in einer ersten Reaktionsstufe unter Anwendung einer Propenbelastung von $\geq 160$ Nl Propen/l Festbettkatalysatorschüttung $1 \cdot h$ so über eine Festbettkatalysatorschüttung 1, die in zwei räumlich aufeinanderfolgenden Reaktionszonen A, B angeordnet ist, wobei die Temperatur der Reaktionszone A 300 bis 370°C und die Temperatur der Reaktionszone B 305 bis 380°C beträgt und gleichzeitig wenigstens 5°C oberhalb der Temperatur der Reaktionszone A liegt und deren Aktivmasse wenigstens ein wenigstens die Elemente Mo, Fe und Bi enthaltendes Multimetalloxid ist, führt, daß sich die Reaktionszone A bis zu einem Umsatz des Propens von 40 bis 80 mol.-% erstreckt und der Propenumsatz bei einmaligem Durchgang durch die Festbettkatalysatorschüttung $1 \geq 90$ mol.-% und die damit einhergehende Selektivität der Acroleinbildung sowie der Acrylsäurenebenproduktbildung (zusammen Selektivität der Wertproduktbildung) zusammengenommen $\geq 90$ mol.-% betragen, und das dabei resultierende Produktgasgemisch, das den molekularen Sauerstoff und das Acrolein in einem molaren Verhältnis $O_2$:$C_3H_4O \geq 0,5$ enthält in einer zweiten Reaktionsstufe so über eine Festbettkatalysatorschüttung 2, die entweder in einer einzigen Reaktionszone C oder in zwei räumlich aufeinanderfolgenden Reaktionszonen D, E angeordnet ist, wobei die Temperatur der Reaktionszone C 230 bis 300°C und die Temperatur der Reaktionszone D 230 bis 280°C und die Temperatur der Reaktionszone E 250 bis 300°C beträgt und gleichzeitig wenigstens 5°C oberhalb der Temperatur der Reaktionszone D liegt und deren Aktivmasse wenigstens ein wenigstens die Elemente Mo und V enthaltendes Multimetalloxid ist, führt, daß der Acroleinumsatz bei einmaligem Durchgang durch die Reaktionszone C bzw. die Reaktionszonen D und E $\geq 90$ mol.-% und die Selektivität der über alle Reaktionszonen bilanzierten Acrylsäurebildung, bezogen auf umgesetztes Propen, $\geq 80$ mol.-% beträgt, wobei die zeitliche Abfolge, in der das Reaktionsgasausgangsgemisch die Reaktionszonen durchströmt, der alphabetischen Abfolge der Reaktionszonen entspricht, dadurch gekennzeichnet, daß sich beide Reaktionsstufen, d.h., sowohl die Festbettkatalysatorschüttungen 1 und 2 als auch die Reaktionszonen A, B und C bzw. A, B, D und E in einem einzigen Vielkontaktrohr-Rohrbündelreaktor befinden.

Unter der Temperatur einer Reaktionszone wird hier die Temperatur des in der Reaktionszone befindlichen Festbettkatalysatorschüttungsanteils bei Ausübung des erfindungsgemäßen Verfahrens in Abwesenheit einer chemischen Reaktion verstanden.

Unter der Belastung einer Katalysatorschüttung mit Reaktant wird hier ganz generell die Menge an Reaktand in Normlitern (= Nl; das Volumen in Liter, das die entsprechende Menge Reaktand bei Normalbedingungen, d.h., bei 25°C und 1 bar einnehmen würde) verstanden, die als Bestandteil des Reaktionsgasausgangsgemisches pro Stunde durch einen Liter an Katalysatorschüttung geführt wird.

Erfindungsgemäß bevorzugt erstreckt sich die Reaktionszone A bis zu einem Propenumsatz von 50 bis 70 mol-% und besonders bevorzugt bis zu einem Propenumsatz von 65 bis 75 mol-%.

Die Temperatur der Reaktionszone B beträgt erfindungsgemäß in vorteilhafter Weise 310 bis 370°C und besonders vorteilhaft 320 bis 370°C.

Ferner liegt die Temperatur der Reaktionszone B bevorzugt wenigstens 10°C oberhalb der Temperatur der Reaktionszone A.

Je höher die Propenbelastung der Festbettkatalysatorschüttung 1 beim erfindungsgemäßen Verfahren gewählt wird, um so größer sollte die Differenz zwischen der Temperatur der Reaktionszone A und der Temperatur der Reaktionszone B gewählt werden. Normalerweise wird die vorgenannte Temperaturdifferenz beim erfindungsgemäßen Verfahren aber nicht mehr als 50°C betragen. D.h., die Differenz zwischen der Temperatur der Reaktionszone A und der Temperatur der Reaktionszone B kann erfindungsgemäß bis zu 20°C, bis zu 25°C, bis zu 30°C, bis zu 40°C, bis zu 45°C oder bis zu 50°C betragen.

In der Regel wird der auf den einfachen Durchgang der ersten Reaktionsstufe bezogene Propenumsatz beim erfingungsgemäßen Verfahren $\geq 92$ mol-% oder $\geq 94$ mol-% betragen. Die Selektivität der Wertproduktbildung wird dabei bei in an sich bekannter Weise geeigneter Katalysatorwahl regelmäßig $\geq 92$ mol-%, oder $\geq 94$ mol-%, häufig $\geq 95$ mol-%, oder $\geq 96$ mol-%, bzw. $\geq 97$ mol-% betragen.

In überraschender Weise gilt das Vorgenannte erfindungsgemäß nicht nur bei Propenbelastungen der Festbettkatalysatorschüttung 1 von $\geq$ 165 Nl/l·h oder von $\geq$ 170 Nl/l·h bzw. $\geq$ 175 Nl/l·h oder $\geq$ 180 Nl/l·h, sondern auch bei Propenbelastungen der Festbettkatalysatorschüttung 1 von $\geq$ 185 Nl/l·h, oder $\geq$ 190 Nl/l·h bzw. $\geq$ 200 Nl/l·h, oder $\geq$ 210 Nl/l·h sowie bei Belastungswerten $\geq$ 220 Nl/l·h, oder $\geq$ 230 Nl/l·h bzw. $\geq$ 240 Nl/l·h oder, $\geq$ 250 Nl/l·h.

Dabei überrascht, daß vorgenannte Werte selbst dann erreichbar sind, wenn das erfindungsgemäß verwendete Inertgas zu $\geq$ 30 Vol.-%, oder zu $\geq$ 40 Vol.-%, oder zu $\geq$ 50 Vol.-%, oder zu $\geq$ 60 Vol.-%, oder zu $\geq$ 70 Vol.-%, oder zu $\geq$ 80 Vol.-%, oder zu $\geq$ 90 vol.-%, oder zu $\geq$ 95 Vol.-% aus molekularem Stickstoff besteht. Bei Propenbelastungen oberhalb von 250 Nl/1·h wird für das erfindungsgemäße Verfahren die Mitverwendung von inerten (inerte Verdünnungsgase sollen generell solche sein, die sich beim einmaligen Durchgang zu weniger als 5 %, bevorzugt zu weniger als 2 % umsetzen) Verdünnungsgasen wie Propan, Ethan, Methan, Pentan, Butan, $CO_2$, CO, Wasserdampf und/oder Edelgasen empfohlen. Selbstverständlich können diese Gase und ihre Gemische aber auch bereits bei geringeren Belastungen mitverwendet oder als alleinige Verdünnungsgase verwendet werden. Ferner überrascht, daß das erfindungsgemäße Verfahren mit einer über die Reaktionszonen A, B betrachtet homogenen, d.h., chemisch einheitlichen, Festbettkatalysatorschüttung 1 durchgeführt werden kann, ohne in nennenswertem Umfang Umsatz- und/oder Selektivitätseinbußen zu erleiden.

Normalerweise wird beim erfindungsgemäßen Verfahren die Propenbelastung der Festbettkatalysatorschüttung 1 den Wert von 600 Nl/l·h nicht überschreiten. In typischer Weise liegen die Propenbelastungen der Festbettkatalysatorschüttung 1 beim erfindungsgemäßen Verfahren ohne nennenswerten Verlust von Umsatz und Selektivität bei Werten $\leq$ 300 Nl/l·h, häufig $\leq$ 250 Nl/l·h.

Der Arbeitsdruck kann beim erfindungsgemäßen Verfahren sowohl unterhalb von Normaldruck (z.B. bis zu 0,5 bar) als auch oberhalb von Normaldruck liegen. Typischerweise wird der Arbeitsdruck bei Werten von 1 bis 5 bar, häufig 1,5 bis 3,5 bar liegen. Normalerweise wird der Reaktionsdruck 100 bar nicht überschreiten.

Das molare Verhältnis von $O_2:C_3H_6$ im Reaktionsgasausgangsgemisch muß erfindungsgemäß $\geq$ 1 betragen. Üblicherweise wird dieses Verhältnis bei Werten $\leq$ 3 liegen. Häufig beträgt das molare Verhältnis von $O_2:C_3H_6$ im Reaktionsgasausgangsgemisch erfindungsgemäß $\geq$ 1,5 und $\leq$ 2,0.

Als Quelle für den im Rahmen des erfindungsgemäßen Verfahrens erforderlichen molekularen Sauerstoff kommt sowohl Luft, als auch an molekularem Stickstoff entreicherte Luft (z.B. $\geq$ 90 Vol.-% $O_2$, $\leq$ 10 Vol.-% $N_2$) in Betracht.

Der Propenanteil im Reaktionsgasausgangsgemisch kann erfindungsgemäß z.B. bei Werten von 4 bis 15 Vol.-%, häufig bei 5 bis 12 Vol.-% bzw. 5 bis 8 Vol.-% liegen (jeweils bezogen auf das Gesamtvolumen).

Häufig wird man das erfindungsgemäße Verfahren bei einem Propen:Sauerstoff:indifferente Gase (einschließlich Wasserdampf)-Volumenverhältnis im Reaktionsgasausgangsgemisch von 1:(1,0 bis 3,0): (5 bis 25), vorzugsweise 1:(1,7 bis 2,3):(10 bis 15) durchführen.

Normalerweise enthält das Reaktionsgasausgangsgemisch neben den genannten Bestandteilen im wesentlichen keine weiteren Komponenten.

Als Festbettkatalysatoren 1 kommen für das erfindungsgemäße Verfahren alle diejenigen in Betracht, deren Aktivmasse wenigstens ein Mo, Bi und Fe enthaltendes Multimetalloxid ist.

D.h., prinzipiell können alle diejenigen Katalysatoren, die in den Schriften DE-C 3338380, DE-A 19902562, EP-A 15565, DE-C 2830765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden, erfindungsgemäß eingesetzt werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1c aus der EP-A 15565 sowie ein in entsprechender Weise herzustellender Katalysator, dessen Aktivmasse jedoch die Zusammensetzung $Mo_{12}Ni_{6,5}Zn_2Fe_2Bi_1P_{0,0065}K_{0,06}O_x \cdot 10SiO_2$ aufweist. Ferner sind hervorzuheben das Beispiel mit der laufenden Nr. 3 aus der DE-A 19855913 (Stöchiometrie: $Mo_{12}Co_7Fe_3Bi_{0,6}K_{0,08}Si_{1,6}O_x$) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese Hohlzylinder eine Geometrie 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen.

Eine Vielzahl der für die Festbettkatalysatorschüttung 1 erfindungsgemäß geeigneten Multimetalloxidaktivmassen läßt sich unter der allgemeinen Formel I

$$Mo_{12}Bi_aFe_bX^1_cX^2_dX^3_eX^4_fO_n \tag{I,}$$

in der die Variablen nachfolgende Bedeutung aufweisen:

$X^1$ = Nickel und/oder Kobalt,
$X^2$ = Thallium, ein Alkalimetall und/oder ein Erdalkalimetall,
$X^3$ = Zink, Phosphor, Arsen, Bor, Antimon, Zinn, Cer, Blei und/oder Wolfram,
$X^4$ = Silicium, Aluminium, Titan und/oder Zirkonium,
a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,

subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden. Selbstverständlich kann erfindungsgemäß auch der Bi, Mo und Fe umfassende Multimetalloxidkatalysator ACS-4 der Fa. Nippon Shokubai verwendet werden.

Prinzipiell können die erfindungsgemäß für die Festbettkatalysatorschüttung 1 geeigneten Aktivmassen, insbesondere jene der allgemeinen Formel I, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidmassen I kann in trockener oder in nasser Form erfolgen. Erfolgt er in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die erfindungsgemäß als Aktivmasse für die Festbettkatalysatoren 1 geeigneten Multimetalloxidmassen, insbesondere jene der allgemeinen Formel I, können für das erfindungsgemäße Verfahren sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht und/oder partiell calcinierten Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Günstige erfindungsgemäß für die Festbettkatalysatorschüttung 1 zu verwendende Multimetalloxidaktivmassen sind ferner Massen der allgemeinen Formel II

$$[Y^1{}_{a'}Y^2{}_{b'}O_{x'}]_p[Y^3{}_{c'}Y^4{}_{d'}Y^5{}_{e'}Y^6{}_{f'}Y^7{}_{g'}Y^2{}_{h'}O_{y'}]_q \qquad \text{(II)},$$

in der die Variablen folgende Bedeutung haben:

$Y^1 =$ Wismut, Tellur, Antimon, Zinn und/oder Kupfer,
$Y^2 =$ Molybdän und/oder Wolfram,
$Y^3 =$ ein Alkalimetall, Thallium und/oder Samarium,
$Y^4 =$ ein Erdalkalimetall, Nickel, Kobalt, Kupfer, Mangan, Zink, Zinn, Cadmium und/oder Quecksilber,
$Y^5 =$ Eisen, Chrom, Cer und/oder Vanadium,
$Y^6 =$ Phosphor, Arsen, Bor und/oder Antimon,
$Y^7 =$ ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

$a' =$ 0,01 bis 8,
$b' =$ 0,1 bis 30,
$c' =$ 0 bis 4,
$d' =$ 0 bis 20,
$e' =$ 0 bis 20,
$f' =$ 0 bis 6,
$g' =$ 0 bis 15,
$h' =$ 8 bis 16,
$x',y' =$ Zahlen, die durch die Wertigkeit und Häufigkeit der von Sauerstoff verschiedenen Elemente in II bestimmt werden und
$p,q =$ Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1{}_{a'}Y^2{}_{b'}O_{x'}$, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte erfindungsgemäße Multimetalloxidmassen II sind solche, in denen $Y^1$ Wismut ist.

Unter diesen werden wiederum jene bevorzugt, die der allgemeinen Formel III

$$[Bi_{a''}Z^2_{b''}O_{x''})_{p''} [Z^2_{12}Z^3_{c''}Z^4_{d''}Fe_{e''}Z^5_{f''}Z^6_{g''}Z^7_{h''}O_{y''}]_{q''}$$ (III)

in der die Varianten folgende Bedeutung haben:

$Z^2 =$    Molybdän und/oder Wolfram,

$Z^3 =$    Nickel und/oder Kobalt,

$Z^4 =$    Thallium, ein Alkalimetall und/oder ein Erdalkalimetall,

$Z^5 =$    Phosphor, Arsen, Bor, Antimon, Zinn, Cer und/oder Blei,

$Z^6 =$    Silicium, Aluminium, Titan und/oder Zirkonium,

$Z^7 =$    Kupfer, Silber und/oder Gold,

$a'' =$    0,1 bis 1,

$b'' =$    0,2 bis 2,

$c'' =$    3 bis 10,

$d'' =$    0,02 bis 2,

$e'' =$    0,01 bis 5, vorzugsweise 0,1 bis 3,

$f'' =$    0 bis 5,

$g'' =$    0 bis 10,

$h'' =$    0 bis 1,

$x'', y'' =$    Zahlen, die durch die Wertigkeit und Häufigkeit der von Sauerstoff verschiedenen Elemente in III bestimmt werden,

$p'', q'' =$    Zahlen, deren Verhältnis $p''/q''$ 0,1 bis 5, vorzugsweise 0,5 bis 2 beträgt,

entsprechen, wobei diejenigen Massen III ganz besonders bevorzugt werden, in denen $Z^2_{b''} = (Wolfram)_{b''}$ und $Z^2_{12} = (Molybdän)_{12}$ ist.

Ferner ist es von Vorteil, wenn wenigstens 25 mol.-% (bevorzugt wenigstens 50 mol.-% und besonders bevorzugt wenigstens 100 mol.-%) des gesamten Anteils $[Y^1_{a'}Y^2_{b'}O_{x'}]_p$ ($[Bi_{a''}Z^2_{b''}O_{x''}]_{p''}$) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgehender, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ $[Bi_{a''}Z^2_{b''}O_{x''}]$ vorliegen, deren Größtdurchmesser im Bereich 1 nm bis 100 μm liegt.

Hinsichtlich der Formgebung gilt bezüglich Multimetalloxidmassen II-Katalysatoren das bei den Multimetalloxidmassen I-Katalysatoren Gesagte.

Im Unterschied zur Festbettkatalysatorschüttung 1 kann sich erfindungsgemäß die Festbettkatalysatorschüttung 2 sowohl in einer eine einheitliche Temperatur aufweisenden Reaktionszone (Reaktionszone C) als auch in zwei voneinander verschiedenen Temperaturen aufweisenden, räumlich aufeinanderfolgenden, Reaktionszonen D, E befinden.

Erfindungsgemäß zweckmäßig beträgt die Temperatur der Reaktionszone C 230 bis 285°C. Ferner beträgt die Temperatur der Reaktionszone D erfindungsgemäß zweckmäßig 245 bis 275°C und die Temperatur der Reaktionszone E liegt erfindungsgemäß vorteilhaft wenigstens 20°C oberhalb der Temperatur der Reaktionszone D und beträgt vorteilhaft 265 bis 295°C.

Je höher die Propenbelastung der Festbettkatalysatorschüttung 1, und damit ganz automatisch die Acroleinbelastung der Festbettkatalysatorschüttung 2, beim erfindungsgemäßen Verfahren gewählt wird, desto vorteilhafter ist eine Anwendung zweier Temperaturzonen D, E anstelle einer Temperaturzone C. Dabei sollte die Differenz zwischen der Temperatur der Reaktionszone D und der Temperatur der Reaktionszone E um so größer gewählt werden, je höher die Reaktandenbelastung gewählt wird.

Normalerweise wird die vorgenannte Temperaturdifferenz beim erfindungsgemäßen Verfahren aber nicht mehr als 40°C betragen. D.h., die Differenz zwischen der Temperatur der Reaktionszone D und der Temperatur der Reaktionszone E kann erfindungsgemäß bis zu 15°C, bis zu 25°C, bis zu 30°C, bis zu 35°C oder bis zu 40°C betragen.

Erfindungsgemäß wesentlich ist, daß der in der zweiten Reaktionsstufe benötigte Sauerstoff bereits im Reaktionsgasausgangsgemisch für die erste Reaktionsstufe enthalten ist.

Außer den in dieser Schrift genannten Bestandteilen enthält das Reaktionsgasausgangsgemisch normalerweise im wesentlichen keine weiteren Komponenten.

Die Acroleinbelastung der Festbettkatalysatorschüttung 2 ist beim erfindungsgemäßen Verfahren eine automatische Konsequenz der in der ersten Reaktionsstufe gewählten Propenbelastung der Festbettkatalysatorschüttung 1 sowie der in der ersten Reaktionsstufe gewählten Reaktionsbedingungen, einschließlich der gewählten Reaktionsgasausgangsgemischzusammensetzung.

In der Regel wird beim erfindungsgemäßen Verfahren die Acroleinbelastung der Festbettkatalysatorschüttung 2 etwa 10 Nl/l·h, häufig etwa 20 bzw. 25 Nl/l·h unterhalb der Propenbelastung der Festbettkatalysatorschüttung 1 liegen. Dies ist primär darauf zurückzuführen, daß in der ersten Reaktionsstufe sowohl Umsatz als auch Selektivität zu Acrolein in der Regel nicht 100 % erreichen.

Bei in an sich bekannter Weise geeigneter Wahl der Festbettkatalysatorschüttung 2 kann beim erfindungsgemäßen Verfahren die über beide Reaktionsstufen bilanzierte Selektivität der Acrylsäurebildung, bezogen auf umgesetztes Propen, selbst bei höchsten Propenbelastungen der Festbettkatalysatorschüttung 1 bei werten $\geq 83$ mol.-%, häufig bei $\geq 85$ mol.-%, oder $\geq 88$ mol.-%, oft bei $\geq 90$ mol.-%, oder $\geq 93$ mol.-% liegen.

Als erfindungsgemäß zu verwendende Festbettkatalysatoren 2 kommen für die gasphasenkatalytische Acroleinoxidation in der zweiten Reaktionsstufe alle diejenigen in Betracht, deren Aktivmasse wenigstens ein Mo und V enthaltendes Multimetalloxid ist. Solchermaßen geeignete Multimetalloxidaktivmassen können beispielsweise der US-A 3 775 474, der US-A 3 954 855, der US-A 3 893 951 und der US-A 4 339 355 entnommen werden. Ferner eignen sich in besonderer Weise die Multimetalloxidaktivmassen der EP-A 427 508, der DE-A 2 909 671, der DE-C 31 51 805, der DE-AS 2 626 887, der DE-A 43 02 991, der EP-A 700 893, der EP-A 714 700 und der DE-A 19 73 6105. Besonders bevorzugt sind in diesem Zusammenhang die beispielhaften Ausführungsformen der EP-A 714 700 sowie der DE-A 19 73 6105.

Eine Vielzahl der für Festbettkatalysatoren 2 geeigneten Multimetalloxidaktivmassen läßt sich unter der allgemeinen Formel IV

$$Mo_{12}V_aX^1{}_bX^2{}_cX^3{}_dX^4{}_eX^5{}_fX^6{}_gO_n \qquad (IV),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle,
$X^5$ = eines oder mehrere Erdalkalimetalle,
$X^6$ = Si, Al, Ti und/oder Zr,
$a$ = 1 bis 6,
$b$ = 0,2 bis 4,
$c$ = 0,5 bis 18,
$d$ = 0 bis 40,
$e$ = 0 bis 2,
$f$ = 0 bis 4,
$g$ = 0 bis 40 und
$n$ = eine Zahl, die durch die Wertigkeit und Häufigkeit der von Sauerstoff verschiedenen Elemente in IV bestimmt wird,

subsummieren.

Bevorzugte Ausführungsformen innerhalb der aktiven Multimetalloxide IV sind jene, die von nachfolgenden Bedeutungen der Variablen der allgemeinen Formel IV erfaßt werden:

$X^1$ = W, Nb, und/oder Cr,
$X^2$ = Cu, Ni, Co, und/oder Fe,
$X^3$ = Sb,
$X^4$ = Na und/oder K,
$X^5$ = Ca, Sr und/oder Ba,
$X^6$ = Si, Al, und/oder Ti,
$a$ = 1,5 bis 5,
$b$ = 0,5 bis 2,
$c$ = 0,5 bis 3,
$d$ = 0 bis 2,
$e$ = 0 bis 0,2,
$f$ = 0 bis 1 und

n = eine Zahl, die durch die Wertigkeit und Häufigkeit der von Sauerstoff verschiedenen Elemente in IV bestimmt wird.

Ganz besonders bevorzugte Multimetalloxide IV sind jedoch jene der allgemeinen Formel V

$$Mo_{12}V_{a'}Y^1_{b'}Y^2_{c'}Y^5_{f'}Y^6_{g'}O_{n'} \qquad (V)$$

mit

Y$^1$ = W und/oder Nb,
Y$^2$ = Cu und/oder Ni,
Y$^5$ = Ca und/oder Sr,
Y$^6$ = Si und/oder Al,
a' = 2 bis 4,
b' = 1 bis 1,5,
c' = 1 bis 3,
f' = 0 bis 0,5,
g' = 0 bis 8 und
n' = eine Zahl, die durch die Wertigkeit und Häufigkeit der von Sauerstoff verschiedenen Elementen in V bestimmt wird.

Die erfindungsgemäß geeigneten Multimetalloxidaktivmassen (IV) sind in sich bekannter, z.B. in der DE-A 4335973 oder in der EP-A 714700 offenbarter, Weise erhältlich.

Prinzipiell können erfindungsgemäß für Festbettkatalysatoren 2 geeignete Multimetalloxidaktivmassen, insbesondere solche der allgemeinen Formel IV, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie H$_2$, NH$_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die erfindungsgemäß als Aktivmassen der Festbettkatalysatoren 2 für die zweite Reaktionsstufe geeigneten Multimetalloxidmassen, insbesondere jene der allgemeinen Formel IV, können für das erfindungsgemäße Verfahren sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Günstige erfindungsgemäß als Festbettkatalysatoren 2 für die zweite Reaktionsstufe erfindungsgemäß zu verwendende Multimetalloxidaktivmassen sind ferner Massen der allgemeinen Formel VI,

$$[D]_p \, [E]_q \qquad\qquad (VI),$$

in der die Variablen folgende Bedeutung haben:

D = $Mo_{12}V_{a"}Z^1_{b"}Z^2_{c"}Z^3_{d"}Z^4_{e"}Z^5_{f"}Z^6_{g"}O_{x"}$ ,
E: = $Z^7_{12}CU_{h"}H_{i"}O_{y"}$ ,
$Z^1$ = W, Nb, Ta, Cr und/oder Ce,
$Z^2$ = Cu, Ni, Co, Fe, Mn und/oder Zn,
$Z^3$ = Sb und/oder Bi,
$Z^4$ = Li, Na, K, Rb, Cs und/oder H,
$Z^5$ = Mg, Ca, Sr und/oder Ba,
$Z^6$ = Si, Al, Ti und/oder Zr,
$Z^7$ = Mo, W, V, Nb und/oder Ta,

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in VI bestimmt werden und
p,q = von Null verschiedene Zahlen, deren Verhältnis p/q 160:1 bis 1:1 beträgt,

und die dadurch erhältlich sind, daß man eine Multimetalloxidmasse E

$$Z^7_{\ 12}Cu_{h"}H_{i"}O_{y"} \qquad\qquad (E),$$

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a''}Z^1_{b''}Z^2_{c''}Z^3_{d''}Z^4_{e''}Z^5_{f''}Z^6_{g''} \qquad (D),$$

enthält (Ausgangsmasse 2), im gewünschten Mengenverhältnis p:q einarbeitet, die dabei gegebenenfalls resultierende wäßrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen VI, bei denen die Einarbeitung der vorgebildeten festen Ausgangsmasse 1 in eine wäßrige Ausgangsmasse 2 bei einer Temperatur ≤ 70°C erfolgt. Eine detaillierte Beschreibung der Herstellung von Multimetalloxidmassen VI-Katalysatoren enthalten z.B. die EP-A 668104, die DE-A 19736105 und die DE-A 19528646.

Hinsichtlich der Formgebung gilt bezüglich Multimetalloxidmassen VI-Katalysatoren das bei den Multimetalloxidmassen IV-Katalysatoren Gesagte.

Selbstredend können beim erfindungsgemäßen Verfahren sowohl Festbettkatalysatorschüttungen 1 als auch Festbettkatalysatorschüttungen 2 verwendet werden, deren volumenspezifische Aktivität in Strömungsrichtung des Reaktionsgasausgangsgemisches kontinuierlich, abrupt oder stufenförmig zunimmt (dies kann z.B. wie in der WO 98/24746, der EP-A 450596, der EP-A 792866 oder wie in der JP-A 91/294239 beschrieben oder auch durch Verdünnung mit Inertmaterial bewirkt werden). Ebenso können erfindungsgemäß neben Stickstoff, Wasserdampf und/oder Kohlenoxiden die in der EP-A 293224 und in der EP-B 257565 empfohlenen inerten Verdünnungsgase (z.B. nur Propan oder nur Methan etc.) eingesetzt werden. Letzteres bei Bedarf auch kombiniert mit einer in Strömungsrichtung des Reaktionsgasgemisches zunehmenden volumenspezifischen Aktivität der Festbettkatalysatorschüttungen.

Erfindungsgemäß kann die Reaktionszone B räumlich unmittelbar an die Reaktionszone C bzw. D anschließen. D.h., die Festbettkatalysatorschüttungen 1 und 2 können erfindungsgemäß unmittelbar aneinandergrenzen. Selbstverständlich können sie aber auch durch Inertmaterialschüttungen und/oder Luft voneinander getrennt sein.

Als trennende Inertmaterialien kommen z.B. die in der DE-C 2830765 sowie die in der EP-A 911313 als Reaktionszonen voneinander trennenden Inertmaterialien in Betracht. Erfindungsgemäß wird eine solche Inertmaterialschüttung der Festbettkatalysatorschüttung 2 zugerechnet, d.h., auf derselben Temperatur wie die Reaktionszone C bzw. D gehalten.

Erfindungswesentlich ist, daß sich beide Reaktionsstufen, d.h., sowohl die Festbettkatalysatorschüttungen 1 und 2 als auch die Reaktionszonen A, B und C bzw. A, B, D und E in einem einzigen Vielkontaktrohr-Rohrbündelreaktor befinden.

D.h., die Verwirklichung des erfindungsgemäßen Verfahrens muß in einem sogenannten Mehrtemperaturzonen-Rohrbündelreaktor erfolgen, wie er am Beispiel von zwei Temperaturzonen in den Schriften DE-C 2830765, DE-C 2513405, der US-A 3147084, der DE-A 2201528, der EP-A 383224 und der DE-A 2903218 offenbart ist.

D.h., die erfindungsgemäß zu verwendenden Festbettkatalysatorschüttungen 1 und 2 sowie eine diese gegebenenfalls voneinander trennende Inertmaterialschüttung befinden sich räumlich aufeinanderfolgend in den Metallrohren eines Rohrbündelreaktors (bei vertikaler Ausrichtung der Reaktionsrohre befindet sich zweckmäßigerweise diejenige der beiden Festbettkatalysatorschüttungen oben, deren Standzeit die geringere ist) und um die Metallrohre werden voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone. D.h., im erfindungsgemäßen Normalfall umströmt ein Salzbad A denjenigen Abschnitt der Rohre (die Reaktionszone A) in welchem sich die oxidative Umsetzung des Propens (beim einfachen Durchgang) bis zum Erreichen eines Umsatzes im Bereich von 40 bis 80 mol.-% vollzieht und ein Salzbad B umströmt den Abschnitt der Rohre (die Reaktionszone B), in welchem sich die oxidative Anschlußumsetzung des Propens (beim einfachen Durchgang) bis zum Erreichen eines Umsatzwertes von wenigstens 90 mol.-% vollzieht. In beiden vorgenannten Rohrabschnitten befindet sich die Festbettkatalysatorschüttung 1. Ein weiteres Salzbad C oder zwei weitere Salzbäder D, E umströmen die Rohrabschnitte, innerhalb derer sich die Festbettkatalysatorschüttung 2 sowie gegebenenfalls die die beiden Festbettkatalysatorschüttungen voneinander trennende Inertmaterialschüttung befindet und in denen sich die Acroleinumsetzung vollzieht.

Üblicherweise liegt der Beginn der Reaktionszone B hinter dem Heißpunktmaximum der Reaktionszone A. In entsprechender Weise liegt der Beginn der Reaktionszone E normalerweise hinter dem Heißpunktmaximum der Reaktionszone D. In der Regel erstreckt sich die Reaktionszone D bis zu einem Umsatz des aus der ersten Reaktionsstufe kommenden Acrolein von 55 bis 85 mol-%, vorzugsweise von 65 bis 80 mol-%.

Jedes der Temperier(Salz-)bäder A, B, C bzw. A, B, D, E kann erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden.

Zweckmäßigerweise wird entweder in allen Reaktionszonen eine Gleichstrom- oder eine Gegenstromführung angewendet. Günstig ist es auch, in der Reaktionszone A eine Gleichstromführung und in der Reaktionszone B eine Gegenstromführung (oder umgekehrt) anzuwenden. Desgleichen gilt für die Reaktionszonen D, E.

Selbstverständlich kann man in allen vorgenannten Fallkonstellationen innerhalb der jeweiligen Reaktionszone der, relativ zu den Reaktionsrohren, erfolgenden Parallelströmung des Temperiermediums noch eine Querströmung überlagern, so daß die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird das Reaktionsgasausgangsgemisch der Festbettkatalysatorschüttung 1 auf die Reaktionstemperatur vorerwärmt zugeführt.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, daß der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Mehrzonenrohrbündelreaktoren die Fließgeschwindigkeit innerhalb der erforderlichen Wärmeaustauschmittelkreisläufen so gewählt, daß die Temperatur des Wärmeaustauschmittels von der Eintrittsstelle in die Reaktionszone bis zur Austrittstelle aus der Reaktionszone (bedingt durch die Exothermie der Reaktion) um 0 bis 15°C ansteigt. D.h., das vorgenannte $\Delta T$ kann erfindungsgemäß 1 bis 10°C, oder 2 bis 8°C oder 3 bis 6°C betragen. Die Eintrittstemperatur des Wärmeaustauschmittels in die Reaktionszone A beträgt erfindungsgemäß normalerweise 300 bis 370°C. Die Eintrittstemperatur des Wärmeaustauschmittels in die Reaktionszone B beträgt erfindungsgemäß normalerweise einerseits 305 bis 380°C und liegt andererseits gleichzeitig wenigstens 5°C oberhalb der Eintrittstemperatur des in die Reaktionszone A eintretenden Wärmeaustauschmittels.

Bevorzugt liegt die Eintrittstemperatur des Wärmeaustauschmittels in die Reaktionszone B wenigstens 10°C oberhalb der Eintrittstemperatur des in die Reaktionszone A eintretenden Wärmeaustauschmittels. Die Differenz zwischen den Eintrittstemperaturen in die Reaktionszone A bzw. B kann erfindungsgemäß somit bis zu 10°C, bis zu 20°C, bis zu 25°C, bis zu 30°C, bis zu 40°C, bis zu 45°C oder bis zu 50°C betragen. Normalerweise wird die vorgenannte Temperaturdifferenz aber nicht mehr als 50°C betragen. Je höher die Propenbelastung der Festbettkatalysatorschüttung 1 beim erfindungsgemäßen Verfahren gewählt wird, um so größer sollte die Differenz zwischen der Eintrittstemperatur des Wärmeaustauschmittels in die Reaktionszone A und der Eintrittstemperatur des Wärmeaustauschmittels in die Reaktionszone B sein.

Mit Vorteil beträgt die Eintrittstemperatur des Wärmeaustauschmittels in die Reaktionszone B erfindungsgemäß 305 bis 375°C und besonders vorteilhaft 305 bis 370°C.

In entsprechender Weise liegt die Eintrittstemperatur des Wärmeaustauschmittels in die Reaktionszone C normalerweise bei 230 bis 300°C, vorzugsweise bei 230 bis 285°C.

Die Eintrittstemperatur des Wärmeaustauschmittels in die Reaktionszone D liegt üblicherweise bei 230 bis 280°C (zweckmäßig bei 245 bis 275°C) und die Eintrittstemperatur des Wärmeaustauschmittels in die Reaktionszone E beträgt im Normalfall 250 bis 300°C (zweckmäßig 265 bis 295°C).

Vorzugsweise liegt die Eintrittstemperatur des Wärmeaustauschmittels in die Reaktionszone E wenigstens 10°C bzw. wenigstens 20°C oberhalb der Eintrittstemperatur des Wärmeaustauschmittels in die Reaktionszone D. Die Differenz zwischen der Eintrittstemperatur des Wärmeaustauschmittels in die Reaktionszone E und in die Reaktionszone D sollte erfindungsgemäß um so größer gewählt werden, je höher die Propenbelastung der Festbettkatalysatorschüttung 1 gewählt wird. Normalerweise beträgt diese Differenz nicht mehr als 50°C. Sie kann jedoch bis zu 15°C, bis zu 25°C, bis zu 30°C, bis zu 35°C oder bis zu 40°C betragen.

Werden die Festbettkatalysatorschüttungen 1 und 2 erfindungsgemäß durch eine Inertmaterialschüttung voneinander getrennt, beträgt die Länge der Inertmaterialschüttung üblicherweise ≤ 30 %, häufig ≤ 20 %, in der Regel jedoch ≥ 10 % der Länge der Festbettkatalysatorschüttung 2.

Solche Inertmaterialien können z.B. Spiralen aus Edelstahl, Ringe aus Steatit oder Kugeln aus Steatit sein. Bei den Inertmaterialien kann es sich aber auch um einzelne in die Reaktionsrohre einzubringende, bis zu 50 cm lange, Spiralen (vgl. EP-B 351167) handeln.

An dieser Stelle sei auch noch einmal darauf hingewiesen, daß für eine Realisierung der erfindungsgemäßen Mehrzonenfahrweise insbesondere auch das in der DE-AS 2201528 beschriebene Prinzip angewendet werden kann, das die Möglichkeit beinhaltet, vom heißeren Wärmeaustauschmittel einer Reaktionszone eine Teilmenge an ein kälteres Wärmeaustauschmittel einer anderen Reaktionszone abzuführen, um gegebenenfalls ein Aufwärmen eines kalten Reaktionsgasausgangsgemisches oder eines kalten Kreisgases zu bewirken.

Ferner kann die Rohrbündelcharakteristik innerhalb einer indivduellen Reaktionszone wie in der EP-A 382098 beschrieben gestaltet werden.

Bemerkenswerterweise bedingt die erfindungsgemäße Verfahrensweise bei vorgegebener Propenbelastung sowie vorgegebenem Umsatz und Selektivität eine deutlich verlangsamte Zunahme des Druckabfalls in der Reaktionszone C bzw. in den Reaktionszonen D, E. Dies wird u.a. darauf zurückgeführt, daß die erfindungsgemäße Gestaltung der ersten Reaktionsstufe ganz offensichtlich die Verflüchtigung des $MoO_3$ mindert, was letztlich erst die Durchführung der erfindungsgemäßen Verfahrensweise in einem einzigen Rohrbündelreaktor ermöglicht.

Erfindungswesentlich ist auch, daß das erfindungsgemäße Verfahren nicht in notwendiger Weise der Mitverwendung einer Zwischenschicht aus Inertmaterial bedarf.

Das erfindungsgemäße Verfahren eignet sich insbesondere für eine kontinuierliche Durchführung.

Bei dem erfindungsgemäßen Verfahren wird keine reine Acrylsäure sondern ein Gemisch erhalten, von dessen Nebenkomponenten die Acrylsäure in an sich bekannter Weise (z.B. rektifikativ und/oder kristallisativ) abgetrennt werden kann. Nicht umgesetztes Acrolein, Propen sowie verwendetes und/oder im Verlauf der Reaktion gebildetes inertes Verdünnungsgas können in die Gasphasenoxidation rückgeführt werden. Bei der erfindungsgemäßen zweistufigen, von Propen ausgehenden Gasphasenoxidation erfolgt die Rückführung zweckmäßigerweise in die Reaktionszone A.

Im übrigen sind in dieser Schrift Umsatz, Selektivität und Verweilzeit, falls nichts anderes erwähnt wird, wie folgt definiert:

$$\text{Umsatz an Edukt (\%)} = \frac{\text{Molzahl umgesetztes Edukt}}{\text{Molzahl eingesetztes Edukt}} \times 100$$

$$\text{Selektivität der Produktbildung} = \frac{\text{Molzahl Edukt umgesetzt zu Produkt}}{\text{Molzahl umgesetztes Edukt}} \times 100$$

$$\text{Verweilzeit (sec.)} = \frac{\text{mit Katalysator gefülltes Leervolumen des Reaktors (l)}}{\text{durchgesetzte Menge Reaktions gasausgangsgemisch (Nl/h)}} \times 3600$$

Beispiel und Vergleichsbeispiel

1) Herstellung eines Festbettkatalysators 1

Bei 60 °C löste man portionsweise 213 kg Ammoniumheptamolybdat in 600 l Wasser und gab unter Rühren 0,97 kg einer 46,8 gew.-%igen Kaliumhydroxidlösung von 20 °C dazu. Es wurde eine zweite Lösung hergestellt, indem man zu 333,7 kg einer Cobaltnitratlösung (12,4 Gew.-% Cobalt) 116,25 kg einer Eisennitratlösung (14,2 Gew.-% Eisen) gab, wobei die Temperatur auf 30 °C gehalten und nach beendeter Zugabe noch 30 Min. gerührt wurde. Bei 60 °C dosierte man 112,3 kg einer Wismutnitratlösung (11,2 Gew.-% Wismut) zu der Eisen-Cobalt-Lösung. Die zweite Lösung wurde innerhalb eines Zeitraums von 30 Min. bei 60 °C zu der Molybdatlösung gegeben. 15 Min. nach beendeter Zugabe wurden 19,16 kg Kieselsol (46,80 Gew.-% $SiO_2$) in die erhaltene Maische gegeben. Danach rührte man 15 Min. Die erhaltene Maische wurde anschließend sprühgetrocknet (Gaseintrittstemperatur = 310°C, Gasaustrittstemperatur = 140°C), wobei ein Pulver mit einem Glühverlust (3 h bei 600 °C) von etwa 30 Gew.-% erhalten wurde.

Die Zusammensetzung der Aktivmasse ist $Mo_{12}Co_7Fe_{2,94}Bi_{0,6}Si_{1,59}X_{0,08}O_x$.

Diese Ausgangsmasse wurde nach dem Sprühtrocknen mit 1,5 Gew.-% Graphit gemischt, kompaktiert und zu zylindrischen Ringen mit 5 mm Außendurchmesser, 3 mm Höhe und 2 mm Lochdurchmesser geformt. Die geformten Ringe wurden in einer Bandcalziniervorrichtung mit acht Kammern unter Luft calziniert. Die Kammern waren auf 160 °C, 200 °C, 230 °C, 270 °C, 380 °C, 430 °C, 500 °C sowie nochmals 500 °C thermostatiert. Die Verweilzeit betrug je 2 h in der ersten bis vierten Kammer und 5 h in der fünften bis achten Kammer. Der erhaltene Katalysator ist der Festbettkatalysator 1.

2) Herstellung eines Festbettkatalysators 2

a) Vorabherstellung einer Phase B der Stöchiometrie $Cu_{1,0}Mo_{0,5}W_{0,5}O_4$

In einem ersten Rührkessel wurden bei etwa 25 °C unter Rühren 620 l Wasser vorgelegt. Anschließend gab man 27,4 kg $(NH4)_6Mo_7O_{24}*4H_2O$ dazu. Nach Erwärmen gab man 40,4 kg $(NH_4)_{10}W_{12}O_{41}*7H_2O$ hinzu und erhitzte das Gemisch unter weiterem Rühren auf 90 °C. Es wurde eine klare gelborange Lösung erhalten (Lösung 1).
Parallel zur Herstellung der Lösung 1 wurden in einem zweiten Rührkessel bei etwa 25 °C unter Rühren 373 l Wasser vorgelegt. Anschließend wurden 61 l 25 gew.-%ige wässrige $NH_3$-Lösung eingerührt. Zu der ammoniakalischen Lösung gab man 61,9 kg Kupfer(II)acetat und rührte das erhaltene Gemisch bis zum Erhalt einer klaren, dunkelblauen Lösung ohne Bodensatz (Lösung 2).
Lösung 2 wurde aus dem zweiten Rührkessel in die Lösung 1 überführt. Anschließend wurde die erhaltene türkisfarbene Maische sprühgetrocknet, wobei die Gaseintrittstemperatur am Sprühturm 250 °C, die Gasaustrittstemperatur 140 °C betrug. Die Sprühtrocknung erfolgte im Gleichstrom.
75 kg des erzeugten Sprühpulvers wurden in einen Kneter dosiert und unter Zugabe von 15 1 Wasser geknetet. Danach wurde das Knetgut in einen Extruder entleert und mittels des Extruders zu Strängen (Länge 1-2 cm; Durchmesser 6 mm) geformt.
Auf einem Bandtrockner wurden die Stränge bei einer Temperatur von 120 °C getrocknet.
Der Katalysatorvorläufer wurde im Drehrohr in kontinuierlicher Fahrweise bei einer Temperatur von 340 °C und einer Verweilzeit von wenigstens 1 h im Luftstrom calziniert. Anschließend wurde der Vorläufer nochmals bei 790 °C calziniert.
Anschließend wurden die Stränge in einer Mühle (Biplexmühle BQ 500) auf einen mittleren Partikeldurchmesser von 3 bis 5 μm gemahlen.
Der resultierende Split wies eine BET-Oberfläche ≤ 1 $m^2/g$ auf. Mittels Röntgenbeugung wurden folgende Phasen festgestellt:

    1. Cu $MoO_4$-III mit Wolframitstruktur,
    2. HT-Kupfermolybdat.

b) Vorabherstellung einer Vorläufermasse für die Phase A der Stöchiometrie $Mo_{10,4}V_3W_{1,2}O_x$

In einem Rührkessel wurden bei etwa 25 °C unter Rühren (70 Upm) 900 l Wasser vorgelegt. Anschließend gab man 122,4 kg $(NH_4)_6Mo_7O_{24}*H_2O$ dazu und erhitzte das Gemisch unter Rühren auf 90 °C. Anschließend wurden 22,2 kg $NH_4VO_3$ (Ammoniummetavanadat) zugegeben. Dann wurden 20,9 kg $(NH_4)_{10}W_{12}O_{41}*7H_2O$ zugegeben; durch 60-minütiges Rühren bei 90 °C erhielt man eine klare orangefarbene Lösung. Ihr pH-Wert betrug 6,2 ± 0,3. Durch Zugabe von Essigsäure wurde der pH-Wert zunächst auf 5,0 ± 0,3 gesenkt und anschließend durch Einrühren von 25 gew.-%iger wässriger $NH_3$-Lösung wieder auf 6,2 ± 0,3 erhöht. Im Ergebnis wurde eine klare, orangefarbene Lösung ohne Bodensatz erhalten. Diese wurde anschließend sprühgetrocknet, wobei die Gaseintrittstemperatur am Sprühturm 240 °C, die Gasaustrittstemperatur 100 °C betrug. Das erhaltene Sprühpulver war hellgelb.

c) Herstellung der Multimetalloxidaktivmasse

75 kg des unter b) erhaltenen Sprühpulvers wurden in einem Trogkneter mit zwei Z-förmigen, horizontal gelagerten Knetarmen vorgelegt. Dann wurden 8,6 1 Essigsäure zugegeben und anschließend durch Zugabe der erforderlichen Menge Wasser eine knetbare Konsistenz eingestellt. Anschließend wurden 12,9 kg der vorab hergestellten Phase B zugegeben und bis zur Homogenität geknetet. Danach wurde das Knetgut in einen Extruder entleert und mittels des Extruders zu Strängen (Länge 1-2 cm; Durchmesser 6 mm) geformt. Auf einem Bandtrockner wurden die Stränge bei einer Temperatur von 120 °C getrocknet.
300 kg der so erzeugten Formlinge wurden auf einen Hordenwagen geladen, der mit 10 Blechen von jeweils 1 m Tiefe und 50 cm Breite bestückt war. Die Bleche waren in zwei Reihen nebeneinander und äquidistant übereinander angeordnet. Die Bleche bestanden aus einem Lochblech mit einem Lochdurchmesser von 3 mm. Der Hordenwagen wurde in einen Hordenofen (Innenmaße: Höhe x Breite x Tiefe: 1,30 m x 1,18 m x 1,10 m) eingeschoben, der im Umluftbetrieb mit einer Umluftmenge von etwa 2500 $m^3/h$ betrieben wurde. Das Prozessgas wurde elektrisch erwärmt; die Temperaturregelung erfolgte über ein Thermoelement im Gasstrom. Die Produkttemperatur wurde durch 20 Thermoelemente überwacht, die etwa in der Mitte der Bleche in der Mitte der Produktschüttung angebracht waren. Die Formlinge wurden so in einer Gasatmosphäre von 1,5 Vol-% $O_2$, 7 Vol-% $NH_3$, Rest $N_2$ folgendermaßen calziniert:
Es wurde mit einer Geschwindigkeit von 5 °C pro min die Temperatur des den Hordenofen durchströmenden Gasgemisches in entsprechenden Heizzonen des Hordenofens von Raumtemperatur auf 325 °C erhöht und 11 h auf dieser Temperatur gehalten. Anschließend wurde der $NH_3$-Gehalt der Gasatmosphäre auf 0 % gesenkt. Danach wurde in entsprechender Weise mit einer Geschwindigkeit von 2,5 °C/min auf 400 °C aufgeheizt und diese Temperatur 80 min aufrecht erhalten. Anschließend wurde auf Raumtemperatur abgekühlt.

Die so erhaltenen calzinierten Stränge wurden in einer Mühle (Biplexmühle BQ 500) zu zweiphasigem Multimetalloxidaktivmassensplit eines mittleren Korndurchmessers von 3 bis 5 µm gemahlen.

d) Katalysatorherstellung

In einer Beschichtungstrommel wurden 70 kg Steatitringe (Außendurchmesser x Höhe x Innendurchmesser = 7 mm x 3 mm x 4 mm) als Katalysatorträger vorgelegt und wie folgt mit Multimetalloxidaktivmassensplit beschichtet:

Mit einer Dosierrinne wurden insgesamt 18,1 kg Multimetalloxidaktivmassensplit in die Beschichtungstrommel gegeben. Parallel dazu wurde ein Glycerin/Wasser-Gemisch (Gewichtsverhältnis Glycerin:Wasser = 1:3) in einer Gesamtmenge von 3,5 1 als Haftflüssigkeit zudosiert.

Abschließend wurde in der Beschichtungstrommel getrocknet. Der Aktivmassenanteil der so erzeugten Schalenkatalysatoren (Festbettkatalysator 2) betrug etwa 20 % ihres Gewichts.

3) Gasphasenkatalytische Oxidation von Propen zu Acrylsäure

a) Die erste Reaktionsstufe

Ein erstes Reaktionsrohr (V2A Stahl; 30 mm Außendurchmesser; 2 mm Wandstärke; 26 mm Innendurchmesser, Länge: 400 cm, sowie ein in der Reaktionsrohrmitte zentriertes Thermorohr (4 mm Außendurchmesser) zur Aufnahme eines Thermoelements, mit dem die Temperatur im Reaktionsrohr ermittelt werden kann) wird von unten nach oben auf einem Kontaktstuhl (0,2 cm Länge) zunächst auf einer Länge von 90 cm mit eine rauhe Oberfläche aufweisenden Steatitringen (3 mm Länge, 5 mm Außendurchmesser, 1,5 mm Wandstärke) als Inertmaterial zum Erwärmen des Reaktionsgasausgangsgemisches und anschließend auf einer Länge von 100 cm mit einem Gemisch aus 30 Gew.-% der vorgenannten Steatitringe und 70 Gew.-% des Festbettkatalysators 1 sowie daran anschließend auf einer Länge von 200 cm nur mit Festbettkatalysator 1 beschickt, bevor die Beschickung auf einer Länge von 10 cm mit den vorgenannten Steatitringen abgeschlossen wird.

In Strömungsrichtung des Reaktionsgasgemisches (das Reaktionsgasgemisch tritt am beim Kontaktstuhl befindlichen Rohrende ein) wird das erste Reaktionsrohr zunächst auf einer Länge von 190 cm mit einem Salzbad A und anschließend auf einer Länge von 210 cm mit einem Salzbad B thermostatisiert (jedes der beiden Salzbäder wird duch eine getrennte Pumpe umgewälzt). Salzbad A definiert eine Reaktionszone A und Salzbad B definiert eine Reaktionszone B.

b) Die zweite Reaktionsstufe

Ein zweites Reaktionsrohr (V2A Stahl; 30 mm Außendurchmesser; 2 mm Wandstärke; 26 mm Innendurchmesser, Länge: 400 cm sowie ein in der Reaktionsrohrmitte zentriertes Thermo-Rohr (4 mm Außendurchmesser) zur Aufnahme eines Thermoelements, mit dem die Temperatur im Reaktionsrohr ermittelt werden kann) befindet sich über ein 5 cm Länge aufweisendes Rohrzwischenstück (V2A Stahl, 115 mm Außendurchmesser, 26 mm Innendurchmesser) mit dem ersten Reaktionsrohr am dem Kontaktstuhl gegenüberliegenden Ende verbunden.

Das Rohrzwischenstück (es dient dem Zweck der Probenentnahme) und das daran anschließende zweite Reaktionsrohr werden zunächst auf einer Gesamtlänge von 95 cm mit eine rauhe Oberfläche aufweisenden Hohlzylindern aus Steatit (3 mm Länge, 7 mm Außendurchmesser, 1,5 mm Wandstärke) zum Temperieren des das erste Reaktionsrohr verlassenden, Acrolein enthaltenden Reaktiongasgemisches und anschließend auf einer Länge von 100 cm mit einem Gemisch aus 20 Gew.-% der vorgenannten Steatitringe sowie 80 Gew.-% des Festbettkatalysators 2 sowie daran anschließend auf einer Länge von 200 cm nur mit Festbettkatalysator 2 beschickt, bevor die Beschickung auf einer Länge von 10 cm mit den vorgenannten Steatitringen als Nachschüttung abgeschlossen wird.

In Strömungsrichtung des Reaktionsgemisches wird das zweite Reaktionsrohr zunächst auf einer Länge von 190 cm mit einem Salzbad D und anschließend auf einer Länge von 210 cm mit einem Salzbad E thermostatisiert (jedes der beiden Salzbäder wird durch eine getrennte Pumpe umgewälzt). Salzbad D definiert eine Reaktionszone D und Salzbad E definiert eine Reaktionszone E. Das 5 cm lange Rohrzwischenstück wird nicht von Salzbad umströmt sondern ist lediglich mittels Mineralwolle in einer Schichtdicke von 5 cm isoliert.

c) Die Gasphasenoxidation

Das vorstehend beschriebene erste Reaktionsrohr wird mit einem Reaktionsgasausgangsgemisch der nachfolgenden Zusammensetzung kontinuierlich beschickt:

6,5 bis 7,0 Vol.-% Propen,
2,0 bis 2,5 Vol.-% $H_2O$,
13,0 bis 13,5 Vol.-% $O_2$,
27 bis 28 Vol.-% Propan und
als Restmenge ad 100 % molekularer Stickstoff.

Dem Produktgasgemisch der ersten Reaktionsstufe wird über das Rohrzwischenstück eine kleine Probe für eine gaschromatographische Analyse entnommen. Im übrigen wird das Produktgasgemisch umittelbar in die nachfolgende Acroleinoxidationsstufe (zu Acrylsäure) geführt (Reaktionsstufe 2). Vom Produktgasgemisch der Acroleinoxidationsstufe wird ebenfalls eine kleine Probe für eine gaschromatographische Analyse entnommen.

Ferner wird die Druckdifferenz $\Delta$p zwischen Eintritt in das zweite Reaktionsrohr und Austritt aus dem zweiten Reaktionsrohr als Funktion der Zeit gemessen.

Die Propenbelastung der Beschickung des ersten Reaktionsrohres wird zu 175 Nl Propen/1·h gewählt.

Die Temperaturen in den Reaktionszonen A, B, D und E werden so gewählt, daß der Umsatz des Propens (bei einfachem Reaktordurchgang) bei Austritt des Gasgemisches aus dem ersten Reaktionsrohr 95 mol-% und der Umsatz des im ersten Reaktionsrohr gebildeten Acroleins (bei einfachem Reaktordurchgang) bei Austritt des Gasgemisches aus dem zweiten Reaktionsrohr 99 mol-% beträgt.

Die Temperatur der Reaktionszone A ist erfindungsgemäß ferner so zu bemessen, daß der Propenumsatz beim Austritt des Gasgemisches aus der Reaktionszone A 40 bis 80 mol-% beträgt.

Als Ergebnis wird folgendes erhalten:

Für den Fall, daß die vorgenannten Bedingungen als Vergleichsbeispiel unter der Randbedingung "Temperatur der Reaktionszone A = Temperatur der Reaktionszone B" und "Temperatur der Reaktionszone D = Temperatur der Reaktionszone E" realisiert werden, nimmt die Druckdifferenz $\Delta$p über die Zeit schneller zu, als wenn die vorgenannten Bedingungen erfindungsgemäß unter der Randbedingung "Temperatur der Reaktionszone A < Temperatur der Reaktionszone B" und "Temperatur der Reaktionszone D = Temperatur der Reaktionszone E" realisiert werden.

## Patentansprüche

1. Verfahren der katalytischen Gasphasenoxidation von Propen zu Acrylsäure, bei dem man ein Propen, molekularen Sauerstoff und wenigstens ein Inertgas enthaltendes Reaktionsgasausgangsgemisch, das den molekularen Sauerstoff und das Propen in einem molaren Verhältnis $O_2:C_3H_6 \geq 1$ enthält, in einer ersten Reaktionsstufe unter Anwendung einer Propenbelastung von $\geq 160$ Nl Propen/l Festbettkatalysatorschüttung 1·h so über eine Festbettkatalysatorschüttung 1, die in zwei räumlich aufeinanderfolgenden Reaktionszonen A, B angeordnet ist, wobei die Temperatur der Reaktionszone A 300 bis 370°C und die Temperatur der Reaktionszone B 305 bis 380°C beträgt und gleichzeitig wenigstens 5°C oberhalb der Temperatur der Reaktionszone A liegt und deren Aktivmasse wenigstens ein wenigstens die Elemente Mo, Fe und Bi enthaltendes Multimetalloxid ist, führt, daß sich die Reaktionszone A bis zu einem Umsatz des Propens von 40 bis 80 mol.-% erstreckt und der Propenumsatz bei einmaligem Durchgang durch die Festbettkatalysatorschüttung 1 $\geq 90$ mol.-% und die damit einhergehende Selektivität der Acroleinbildung sowie der Acrylsäurenebenproduktbildung zusammengenommen $\geq 90$ mol.-% betragen, und das dabei resultierende Produktgasgemisch, das den molekularen Sauerstoff und das Acrolein in einem molaren Verhältnis $O_2:C_3H_4O \geq 0,5$ enthält, in einer zweiten Reaktionsstufe so über eine Festbettkatalysatorschüttung 2, die entweder in einer einzigen Reaktionszone C oder in zwei räumlich aufeinanderfolgenden Reaktionszonen D, E angeordnet ist, wobei die Temperatur der Reaktionszone C 230 bis 300°C und die Temperatur der Reaktionszone D 230 bis 280°C und die Temperatur der Reaktionszone E 250 bis 300°C beträgt und gleichzeitig wenigstens 5°C oberhalb der Temperatur der Reaktionszone D liegt und deren Aktivmasse wenigstens ein wenigstens die Elemente Mo und V enthaltendes Multimetalloxid ist, führt, daß der Acroleinumsatz bei einmaligem Durchgang durch die Reaktionszone C bzw. die Reaktionszonen D und E $\geq 90$ mol.-% und die Selektivität der über alle Reaktionszonen bilanzierten Acrylsäurebildung, bezogen auf umgesetztes Propen, $\geq 80$ mol.-% beträgt, wobei die zeitliche Abfolge, in der das Reaktionsgasausgangsgemisch die Reaktionszonen durchströmt der alphabetischen Abfolge der Reaktionszonen entspricht, **dadurch gekennzeichnet, daß** sich beide Reaktionsstufen, d.h., sowohl die Festbettkatalysatorschüttungen 1 und 2 als auch die Reaktionszonen A, B und C bzw. A, B, D und E in einem einzigen Vielkontaktrohr-Rohrbündelreaktor befinden.

2. Verfahren nach Anspruch 1, **dadurch gekennzeichnet, daß** sich die Reaktionszone A bis zu einem Propenumsatz von 50 bis 70 mol.-% erstreckt.

3. Verfahren nach Anspruch 1, **dadurch gekennzeichnet, daß** sich die Reaktionszone A bis zu einem Propenumsatz von 65 bis 75 mol.-% erstreckt.

4. Verfahren nach einem der Ansprüche 1 bis 3, **dadurch gekennzeichnet, daß** die Temperatur der Reaktionszone B wenigstens 10°C oberhalb der Temperatur der Reaktionszone A liegt.

5. Verfahren nach einem der Ansprüche 1 bis 4, **dadurch gekennzeichnet, daß** die Propenbelastung der Festbettkatalysatorschüttung 1 ≥ 180 Nl/l·h beträgt.

6. Verfahren nach einem der Ansprüche 1 bis 5, **dadurch gekennzeichnet, daß** die Aktivmasse der Festbettkatalysatorschüttung 1 wenigstens ein Multimetalloxid der allgemeinen Formel I

$$MO_{12}Bi_aFe_bX_c^1X_d^2X_e^3X_g^4O_n \qquad (I),$$

in der die Variablen nachfolgende Bedeutung aufweisen:

$X^1$ = Nickel und/oder Kobalt,
$X^2$ = Thallium, ein Alkalimetall und/oder ein Erdalkalimetall,
$X^3$ = Zink, Phosphor, Arsen, Bor, Antimon, Zinn, Cer, Blei und/oder Wolfram,
$X^4$ = Silicium, Aluminium, Titan und/oder Zirkonium,

a = 0,5 bis 5,
b = 0,01 bis 5,
c = 0 bis 10,
d = 0 bis 2,
e = 0 bis 8,
f = 0 bis 10 und
n = eine Zahl, die durch die Wertigkeit und Häufigkeit der von Sauerstoff verschiedenen Elemente in I bestimmt wird,

ist.

7. Verfahren nach einem der Ansprüche 1 bis 6, **dadurch gekennzeichnet, daß** die Temperatur der Reaktionszone E wenigstens 20°C oberhalb der Temperatur der Reaktionszone D liegt.

8. Verfahren nach einem der Ansprüche 1 bis 7, **dadurch gekennzeichnet, daß** die über beide Reaktionsstufen bilanzierte Selektivität der Acrylsäurebildung, bezogen auf umgesetztes Propen, ≥ 90 mol.-% beträgt.

9. Verfahren nach einem der Ansprüche 1 bis 8, **dadurch gekennzeichnet, daß** die Aktivmasse der Festbettkatalysatorschüttung 2 wenigstens ein Multimetalloxid der allgemeinen Formel IV

$$Mo_{12}V_aX^1_{\ b}X^2_{\ c}X^3_{\ d}X^4_{\ e}X^5_{\ f}X^6_{\ g}O_n \qquad (IV),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle,
$X^5$ = eines oder mehrere Erdalkalimetalle,
$X^6$ = Si, Al, Ti und/oder Zr,
a = 1 bis 6,
b = 0,2 bis 4,
c = 0,5 bis 18,
d = 0 bis 40,

e =0 bis 2,

f =0 bis 4,

g =0 bis 40 und

n =eine Zahl, die durch die Wertigkeit und Häufigkeit der von Sauerstoff verschiedenen Elemente in IV bestimmt wird,

ist.

## Claims

1. A process for the catalytic gas-phase oxidation of propene to acrylic acid, in which a reaction gas starting mixture which comprises propene, molecular oxygen and at least one inert gas and contains the molecular oxygen and the propene in a molar $O_2$: $C_3H_6$ ratio of $\geq 1$ is passed, in a first reaction stage, using a propene loading of $\geq 160$ l (S.T.P.) of propene/l of fixed catalyst bed 1 per h, over a fixed catalyst bed 1 which is arranged in two spatially successive reaction zones A, B, the temperature of the reaction zone A being from 300 to 370°C and the temperature of the reaction zone B being from 305 to 380°C and simultaneously at least 5°C above the temperature of the reaction zone A and the active material of which being at least one multimetal oxide containing at least the elements Mo, Fe and Bi, in such a way that the reaction zone A extends to a propene conversion of from 40 to 80 mol% and the propene conversion during a single pass through the fixed catalyst bed I is $\geq 90$ mol% and the associated selectivity of the acrolein formation and of the acrylic acid byproduct formation together are $\geq 90$ mol%, and the resulting product gas mixture, which contains the molecular oxygen and the acrolein in a molar $O_2$: $C_3H_4O$ ratio of $\geq 0.5$, is passed, in a second reaction stage, over a fixed catalyst bed 2 which is arranged either in a single reaction zone C or in two spatially successive reaction zones D, E, the temperature of the reaction zone C being from 230 to 300°C and the temperature of the reaction zone D being from 230 to 280°C and the temperature of the reaction zone E being from 250 to 300°C and simultaneously at least 5°C above the temperature of the reaction zone D and the active material of which being at least one multimetal oxide containing at least the elements Mo and V, in such a way that the acrolein conversion during a single pass through the reaction zone C or the reaction zones D and E is $\geq 90$ mol% and the selectivity of the acrylic acid formation balanced over all reaction zones and based on propene converted is $\geq 80$ mol%, the sequence in which the reaction gas starting mixture flows through the reaction zones corresponding to the alphabetic sequence of the reaction zones, wherein both reaction stages, i.e. both the fixed catalyst beds 1 and 2 and the reaction zones A, B and C or A, B, D and E are present in a single tube-bundle reactor comprising a plurality of catalyst tubes.

2. A process as claimed in claim 1, wherein the reaction zone A extends to a propene conversion of from 50 to 70 mol%.

3. A process as claimed in claim 1, wherein the reaction zone A extends to a propene conversion of from 65 to 75 mol%.

4. A process as claimed in any of claims 1 to 3, wherein the temperature of the reaction zone B is at least 10°C above the temperature of the reaction zone A.

5. A process as claimed in any of claims 1 to 4, wherein the propene loading of the fixed catalyst bed 1 is $\geq 180$ l (S. T.P.)/l·h.

6. A process as claimed in any of claims 1 to 5, wherein the active material of the fixed catalyst bed 1 is at least one multimetal oxide of the formula I

$$MO_{12}Bi_aFe_bX^1_cX^2_dX^3_eX^4_fO_n \tag{I},$$

where

$X^1$ is nickel and/or cobalt,

$X^2$ is thallium, an alkali metal and/or an alkaline earth metal,

$X^3$ is zinc, phosphorus, arsenic, boron, antimony, tin, cerium, lead and/or tungsten,

$X^4$ is     silicon, aluminum, titanium and/or zirconium,

a is     from 0.5 to 5,
b is     from 0.01 to 5,
c is     from 0 to 10,
d is     from 0 to 2,
e is     from 0 to 8,
f is     from 0 to 10 and
n is     a number which is determined by the valency and frequency of the elements other than oxygen in I.

**7.** A process as claimed in any of claims 1 to 6, wherein the temperature of the reaction zone E is at least 20°C above the temperature of the reaction zone D.

**8.** A process as claimed in any of claims 1 to 7, wherein the selectivity of the acrylic acid formation, balanced over both reaction stages and based on propene converted, is ≥90 mol%.

**9.** A process as claimed in any of claims 1 to 8, wherein the active material of the fixed catalyst bed 2 is at least one multimetal oxide of the formula IV

$$Mo_{12}V_aX^1{}_bX^2{}_cX^3{}_dX^4{}_eX^5{}_fX^6{}_gO_n \qquad\qquad (IV),$$

where

$X^1$ is     W, Nb, Ta, Cr and/or Ce,
$X^2$ is     Cu, Ni, Co, Fe, Mn and/or Zn,
$X^3$ is     Sb and/or Bi,
$X^4$ is     one or more alkali metals,
$X^5$ is     one or more alkaline earth metals,
$X^6$ is     Si, Al, Ti and/or Zr,
a is     from 1 to 6,
b is     from 0.2 to 4,
c is     from 0.5 to 18,
d is     from 0 to 40,
e is     from 0 to 2,
f is     from 0 to 4,
g is     from 0 to 40 and
n is     a number which is determined by the valency and frequency of the elements other than oxygen in IV.

**Revendications**

**1.** Procédé d'oxydation catalytique en phase gazeuse de propène en acide acrylique, dans lequel un mélange réactionnel gazeux de départ, contenant du propène, de l'oxygène moléculaire et au moins un gaz inerte, et qui contient l'oxygène moléculaire et le propène en une proportion molaire $O_2 : C_3H_6 \geq 1$ est, dans une première étape de réaction et en utilisant une charge de propène ≥ 160 Nl de propène / l de remplissage de catalyseur à lit fixe 1.h, guidé sur un remplissage de catalyseur à lit fixe 1 agencé en deux zones de réaction A, B spatialement successives, la température de la zone de réaction A étant de 300 à 370°C et la température de la zone de réaction B étant de 305 à 380°C et étant en même temps supérieure d'au moins 5°C à la température de la zone de réaction A et dont la masse active est un oxyde multimétallique contenant au moins les éléments Mo, Fe et Bi, de telle manière que la zone de réaction A s'étende jusqu'à un taux de conversion du propène de 40 à 80% molaires et que la conversion du propène pour un simple passage dans le remplissage de catalyseur à lit fixe 1 soit ≥ 90% molaires et que la sélectivité concomitante de la formation d'acroléine ainsi que de la formation des sous-produits d'acide acrylique soit au total ≥ 90% molaires, et le mélange de produits gazeux résultant, qui contient l'oxygène moléculaire et l'acroléine en une proportion molaire $O_2 : C_3H_4O \geq 0,5$ est, dans une deuxième étape de réaction, guidé sur un remplissage de catalyseur à lit fixe 2, qui est disposé soit en une unique zone de réaction C, soit en deux zones de réaction D, E spatialement successives, où la température de la zone de réaction C est de 230 à 300°C et la température de la zone de réaction D est de 230 à 280°C et la température de la zone de réaction E est de 250 à 300°C, et est en même temps supérieure d'au moins 5°C à la température de la zone de réaction D, et dont la masse active est un oxyde multimétallique contenant au moins les éléments Mo et V, de manière que la conversion de l'acroléine, pour un simple passage dans la zone de réaction C ou les zones de réaction D et E soit ≥ 90% molaires et que la sélectivité de la formation d'acide acrylique cumulée sur toutes les zones de réaction, par rapport au propène mis en oeuvre, soit ≥ 80% molaires, la séquence temporelle selon laquelle le mélange réactionnel gazeux de départ traverses les zones de réaction correspondant à l'ordre alphabétique des zones de réaction, **caractérisé en ce que** les deux étapes de réaction, c'est-à-dire tant les deux remplissages de catalyseur à lit fixe 1 et 2 que les zones de réaction A, B et C ou A, B, D et E se trouvent dans un seul réacteur à faisceau de tubes à nombreux tubes de contact.

2. Procédé selon la revendication 1, **caractérisé en ce que** la zone de réaction A s'étend jusqu'à une conversion du propène de 50 à 70% molaires.

3. Procédé selon la revendication 1, **caractérisé en ce que** la zone de réaction A s'étend jusqu'à une conversion du propène de 65 à 75% molaires.

4. Procédé selon l'une quelconque des revendications 1 à 3, **caractérisé en ce que** la température de la zone de réaction B est d'au moins 10°C supérieure à la température de la zone de réaction A.

5. Procédé selon l'une quelconque des revendications 1 à 4, **caractérisé en ce que** la charge de propène du remplissage de catalyseur à lit fixe 1 est ≥ 180 Nl/l·h.

6. Procédé selon l'une quelconque des revendications 1 à 5, **caractérisé en ce que** la masse active du remplissage de catalyseur à lit fixe 1 est au moins un oxyde multimétallique de la formule I

$$Mo_{12}Bi_aFe_bX^1{}_cX^2{}_dX^3{}_eX^4{}_fO_n \qquad (I)$$

dans laquelle les variables ont la signification suivante:

$X^1$ = du nickel et/ou du cobalt,
$X^2$ = du thallium, un métal alcalin et/ou un métal alcalino-terreux,
$X^3$ = du zinc, du phosphore, de l'arsenic, du bore, de l'antimoine, de l'étain, du cérium, du plomb et/ou du tungstène,
$X^4$ = du silicium, de l'aluminium, du titane et/ou du zirconium,

a = 0,5 à 5,
b = 0,01 à 5,
c = 0 à 10,
d = 0 à 2,
e = 0 à 8,
f = 0 à 10 et
n = un nombre déterminé par la valence et l'occurrence des éléments différents de l'oxygène dans I.

7. Procédé selon l'une quelconque des revendications 1 à 6, **caractérisé en ce que** la température de la zone de réaction E est d'au moins 20°C supérieure à la température de la zone de réaction D.

8. Procédé selon l'une quelconque des revendications 1 à 7, **caractérisé en ce que** la sélectivité, cumulée sur les deux étapes de réaction, de la formation d'acide acrylique, est ≥ 90% molaires, par rapport au propène converti.

9. Procédé selon l'une quelconque des revendications 1 à 8, **caractérisé en ce que** la masse active du remplissage de catalyseur à lit fixe 2 est au moins un oxyde multimétallique de la formule générale IV

$$MO_{12}V_aX^1{}_bX^2{}_cX^3{}_dX^4{}_eX^5{}_fX^6{}_gO_n \qquad (IV)$$

dans laquelle les variables ont la signification suivante:

$X^1$ =      W, Nb, Ta, Cr et/ou Ce,
$X^2$ =      Cu, Ni, Co, Fe, Mn et/ou Zn,
$X^3$ =      Sb et/ou Bi,
$X^4$ =      un ou plusieurs métaux alcalins,
$X^5$ =      un ou plusieurs métaux alcalino-terreux,
$X^6$ =      Si, Al, Ti et/ou Zr,
a =      1 à 6,
b =      0,2 à 4,
c =      0,5 à 18,
d =      0 à 40,
e =      0 à 2,
f =      0 à 4,
g =      0 à 40 et
n =      un nombre déterminé par la valence et l'occurrence des éléments différents de l'oxygène dans IV.